# EUROPÄISCHE PATENTANMELDUNG

(11) **EP 3 932 173 A1**
(43) Veröffentlichungstag der Anmeldung: **05.01.2022**
(21) Anmeldenummer: 21176252.1
(22) Anmeldetag: 27.05.2021
(51) Int. Cl.: A01D 41/127, G01N 21/00

(54) **LANDWIRTSCHAFTLICHE ERNTEMASCHINE**

(30) Priorität: 29.06.2020 DE 102020117069
(71) Anmelder: CLAAS Selbstfahrende Erntemaschinen GmbH, 33428 Harsewinkel (DE)
(72) Erfinder: Fischer, Frédéric, 59759 Arnsberg (DE); Barther, Marvin, 33803 Steinhagen (DE); Heufekes, Maik, 48145 Münster (DE); Neitemeier, Dennis, 59510 Lippetal (DE); Witte, Johann, 58730 Fröndenberg (DE)
(74) Vertreter: CLAAS Gruppe

(57) **Zusammenfassung**

Die Erfindung betrifft eine landwirtschaftliche Erntemaschine mit mindestens einem Arbeitsaggregat (2), wobei Erntegut (4) im Betrieb der Erntemaschine entlang eines Ernteguttransportweges (5) durch die Erntemaschine in einem Erntegutstrom transportiert wird, mit einer Kontrollanordnung (8), die ein an dem Ernteguttransportweg (5) angeordnetes optisches Messsystem (9) zur Analyse des Ernteguts (4) aufweist, wobei das optische Messsystem (9) einen ersten optischen Sensor (10) aufweist, wobei das optische Messsystem (9) in einer Messroutine ortsaufgelöste Bilddaten des ersten optischen Sensors (10) in einem ersten Ausschnitt (A₁) aufnimmt, wobei die Erntemaschine eine Auswerteeinrichtung (12) zur Ermittlung eines Erntegutparameters aufweist. Es wird vorgeschlagen, dass das optische Messsystem (9) einen zweiten optischen Sensor (13) aufweist, dass das optische Messsystem (9) in der Messroutine Bilddaten des zweiten optischen Sensors (13) in einem zweiten Ausschnitt (A₂) aufnimmt, dass der erste und der zweite Ausschnitt (A_{1,} A₂) zumindest teilweise in einem Überlappungsausschnitt (U) überlappen und dass die Auswerteeinrichtung (12) in einer Analyseroutine die Bilddaten des ersten optischen Sensors (10) zu dem Überlappungsausschnitt (U) und des zweiten optischen Sensors (13) zu dem Überlappungsausschnitt (U) miteinander korreliert und so den Erntegutparameter ermittelt.

## Beschreibung

Die Erfindung betrifft eine landwirtschaftliche Erntemaschine gemäß dem Oberbegriff von Anspruch 1 sowie ein Verfahren zum Betrieb einer landwirtschaftlichen Erntemaschine gemäß Anspruch 12.

Landwirtschaftliche Erntemaschinen, zu denen etwa Mähdrescher und Feldhäcksler zählen, ernten einen Feldbestand von einem Feld ab und verarbeiten das so gewonnene Erntegut mittels einer Reihe von Arbeitsaggregaten. Grundsätzlich kann bereits der Feldbestand unterschiedliche Qualitäten aufweisen. Teilweise ist die Qualität des Ernteguts jedoch auch beim Erntevorgang noch beeinflussbar. Insbesondere der Separierung von Kornbestandteilen und Nicht-Kornbestandteilen kommt hohe Bedeutung zu. Es ist also einerseits für die direkte Korrektur des Erntevorgangs und andererseits als Information und zur Dokumentation wichtig, die Qualität oder ganz allgemein Erntegutparameter des Ernteguts zu ermitteln.

Die bekannte landwirtschaftliche Erntemaschine (EP 2 826 356 A1), von der die Erfindung ausgeht, transportiert das Erntegut in einem Erntegutstrom im Betrieb der Erntemaschine entlang eines Ernteguttransportweges durch die Erntemaschine. Sie weist eine Kontrollanordnung auf, die ein an dem Ernteguttransportweg angeordnetes optisches Messsystem zur Analyse der Zusammensetzung und/oder von Inhaltsstoffen des Ernteguts aufweist. Das optische Messsystem weist einen optischen Sensor zur ortsaufgelösten Aufnahme von sichtbarem Licht aus einem sichtbaren Wellenlängenbereich in einem Sichtfeld auf. In einer Messroutine nimmt das optische Messsystem ortsaufgelöste Bilddaten des optischen Sensors in dem sichtbaren Wellenlängenbereich auf, die Erntegut in einem Ausschnitt des Erntegutstroms abbilden. Bei der bekannten landwirtschaftlichen Erntemaschine ist der optische Sensor eine handelsübliche Kamera. Weiterhin weist die Erntemaschine eine Auswerteeinrichtung zur Ermittlung eines Erntegutparameters anhand von Bilddaten des optischen Sensors auf.

Es ist grundsätzlich auch bekannt, optische Sensoren oder andere Sensoren einzusetzen, um insbesondere Erntegutparameter zu ermitteln, die im sichtbaren Bereich nicht optisch ermittelt werden können.

Es ist jedoch so, dass das Erntegut weder örtlich noch zeitlich homogen ist. Es besteht daher ein Bedarf, die bekannten Systeme hinsichtlich ihrer Ortsauflösung und/oder ihrer Zeitauflösung zu optimieren.

Der Erfindung liegt das Problem zugrunde, die bekannte landwirtschaftliche Erntemaschine derart auszugestalten und weiterzubilden, dass ganz allgemein die Präzision der Ermittlung der Erntegutparameter optimiert wird.

Das obige Problem wird bei einer landwirtschaftlichen Erntemaschine gemäß dem Oberbegriff von Anspruch 1 durch die Merkmale des kennzeichnenden Teils von Anspruch 1 gelöst.

Wesentlich ist die grundsätzliche Überlegung, eine, insbesondere kamerabasierte, Messung im sichtbaren Wellenlängenbereich örtlich mit einer weiteren optischen Messung zu kombinieren. Diese weitere optische Messung findet dabei im nicht-sichtbaren Wellenlängenbereich statt. Indem zumindest teilweise ein übereinstimmender Überlappungsausschnitt des Ernteguts analysiert wird, können die Daten der beiden optischen Sensoren nicht mehr nur zur Ermittlung getrennter Erntegutparameter verwendet werden, sondern zur gemeinsamen Ermittlung eines Erntegutparameters.

Im Einzelnen wird vorgeschlagen, dass das optische Messsystem einen zweiten optischen Sensor zur Aufnahme von nicht-sichtbarem Licht aus einem nicht-sichtbaren Wellenlängenbereich in einem zweiten Sichtfeld aufweist, dass das optische Messsystem in der Messroutine Bilddaten des zweiten optischen Sensors in dem nicht-sichtbaren Wellenlängenbereich aufnimmt, die Erntegut in einem zweiten Ausschnitt des Erntegutstroms abbilden, dass der erste und der zweite Ausschnitt zumindest teilweise in einem Überlappungsausschnitt überlappen und dass die Auswerteeinrichtung in einer Analyseroutine die Bilddaten des ersten optischen Sensors zu dem Überlappungsausschnitt und des zweiten optischen Sensors zu dem Überlappungsausschnitt miteinander korreliert und so den Erntegutparameter ermittelt.

Bei der besonders bevorzugten Ausgestaltung gemäß Anspruch 2 ist der erste optische Sensor als Kamera, insbesondere als RGB-Kamera, ausgestaltet und/oder der zweite optische Sensor als, insbesondere Nah-Infrarot-, Spektrometer ausgestaltet.

Dabei ist es so, dass insbesondere Nah-Infrarot-Daten besonders schwankungsanfällig sind. Diese Schwankungen basieren jedoch häufig nicht auf Änderungen des Erntegutparameters, sondern auf Änderungen der Messbedingungen, anderer Erntegutparameter und dergleichen. Viele dieser Schwankungen wiederum sind im sichtbaren Wellenlängenbereich detektierbar. Häufig sind diese Schwankungen außerdem lokal sehr begrenzt. Durch den vorschlagsgemäßen Überlappungsausschnitt lässt sich die Ermittlung einiger Erntegutparameter nun deutlich präzisieren oder teilweise erst ermöglichen.

Gemäß Anspruch 3 kann das optische Messsystem ein Gehäuse und ein lichtdurchlässiges Fenster aufweisen. Der erste und der zweite optische Sensor sind in dem Gehäuse angeordnet. Durch das gemeinsame Gehäuse und eine weiter bevorzugte Strahlführung des zu messenden Lichts in dem Gehäuse, kann das Überlappen des ersten und des zweiten Ausschnitts robust und besonders präzise sichergestellt werden.

Die Ermittlung des Erntegutparameters kann gemäß Anspruch 4 dadurch geschehen, dass die Auswerteeinrichtung aus den Bilddaten des zweiten Sensors den Erntegutparameter ermittelt und mittels der Bilddaten des ersten Sensors korrigiert. Anspruch 5 gibt die bevorzugt ermittelbaren Erntegutparameter an.

Gemäß Anspruch 6 kann der erste optische Sensor vorzugsweise ortsaufgelöst zwischen mindestens zwei sichtbaren Wellenlängenbereichen unterscheiden. Dies kann durch die bereits angesprochene RGB-Kamera geschehen, andere Ausgestaltungen sind jedoch ebenso bevorzugt. So wird dem Umstand Rechnung getragen, dass viele Informationen über das Erntegut in unterschiedlichen Farbbereichen detektierbar sind.

Anspruch 7 betrifft bevorzugte Ausgestaltungen der Hardware des ersten optischen Sensors.

Die Ansprüche 8 und 9 betreffen bevorzugte Ausgestaltungen des zweiten optischen Sensors hinsichtlich seiner Ortsauflösung und bevorzugt detektierbare Wellenlängenbereiche.

Anspruch 10 betrifft den bevorzugten Fall, dass der erste Ausschnitt und der zweite Ausschnitt einander zumindest teilweise überdecken. Bei dieser Überdeckung existiert vorzugsweise keine zeitliche Komponente, sie betrifft also ein und denselben Ort. So können Einflüsse durch dynamische Veränderungen des Ernteguts oder Erntegutstroms minimiert werden.

Anspruch 11 betrifft bevorzugte Ausgestaltungen des lichtdurchlässigen Fensters und dessen Anordnung gegenüber der landwirtschaftlichen Erntemaschine im Übrigen und die bevorzugte Anordnung des optischen Messsystems und/oder seiner Teile relativ zur landwirtschaftlichen Erntemaschine im Übrigen.

Nach einer weiteren Lehre gemäß Anspruch 12, der eigenständige Bedeutung zukommt, wird ein Verfahren zum Betrieb einer vorschlagsgemäßen landwirtschaftlichen Erntemaschine beansprucht. Auf alle Ausführungen zu der vorschlagsgemäßen landwirtschaftlichen Erntemaschine darf verwiesen werden.

Im Folgenden wird die Erfindung anhand einer lediglich ein Ausführungsbeispiel darstellenden Zeichnung näher erläutert. In der Zeichnung zeigt
- Fig. 1: eine schematische Seitenansicht eines Mähdreschers als vorschlagsgemäße landwirtschaftliche Erntemaschine und
- Fig. 2: eine schematische Seitenansicht eines Kornelevators des Mähdreschers der Fig. 1 mit einem vorschlagsgemäßen optischen Messsystem.

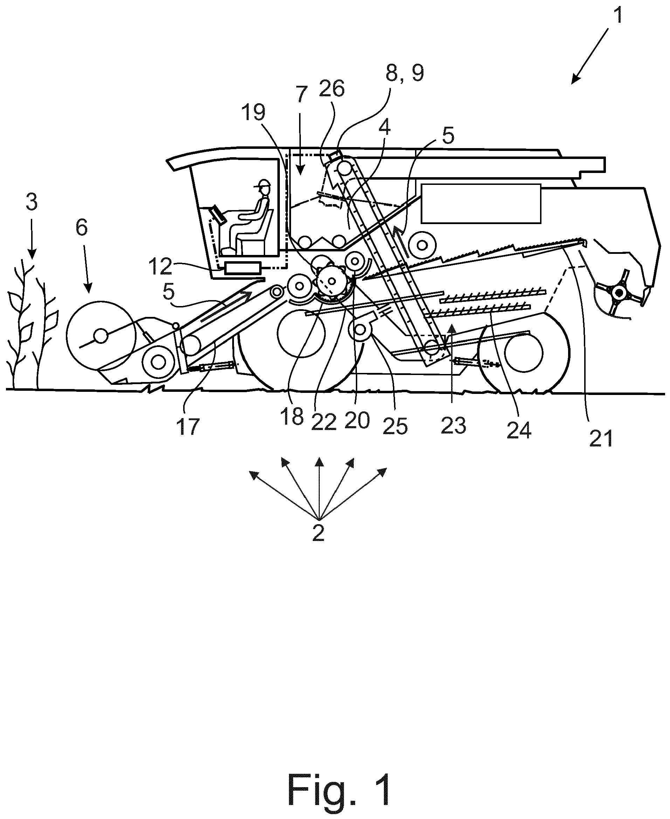

Die in Fig. 1 dargestellte landwirtschaftliche Erntemaschine, bei der es sich hier vorzugsweise um einen Mähdrescher 1 handelt, weist mindestens ein Arbeitsaggregat 2 zum Abernten eines Feldbestandes 3 und zum Verarbeiten von Erntegut 4 des Feldbestandes 3 auf. Eine weitere bevorzugte Erntemaschine ist ein Feldhäcksler. Im Betrieb der Erntemaschine wird das Erntegut 4 in einem Erntegutstrom entlang eines Ernteguttransportweges 5 durch die Erntemaschine transportiert.

Während des Transports durch die Erntemaschine bildet das Erntegut 4 einen Erntegutstrom. Unter dem Begriff "Erntegutstrom" ist vorliegend der Strom der zu verarbeitenden Pflanzenteile des Feldbestandes 3 auf dem Ernteguttransportweg 5 zu verstehen. Dieser Ernteguttransportweg 5 beginnt hier und vorzugsweise, speziell bei einem Mähdrescher 1, bei einem Schneidwerk 6 und geht jedenfalls bis zum Korntank 7. Der Erntegutstrom kann sich in einen Haupterntegutstrom und kleinere Teil-Erntegutströme unterteilen. Der Begriff "Haupterntegutstrom" bezeichnet dann denjenigen Teil des Erntegutstroms, der den überwiegenden Teil des Ernteguts 4, bezogen auf den gesamten Ernteguttransportweg 5, beinhaltet. Insbesondere zu Analysezwecken abzweigende Teil-Erntegutströme in kleinerem Umfang sind nicht umfasst.

Die landwirtschaftliche Erntemaschine weist eine Kontrollanordnung 8 auf, die ein an dem Ernteguttransportweg 5 angeordnetes optisches Messsystem 9 zur Analyse des Ernteguts 4 aufweist. Die Analyse des Ernteguts umfasst eine Analyse der Zusammensetzung (Anteil an unbeschädigtem Korn, Anteil an Bruchkorn, Anteil an Nicht-Korn etc.) des Ernteguts im Erntegutstrom und/oder eine Analyse der Inhaltsstoffe (Feuchtigkeitsgehalt, Eiweißgehalt, Stärkegehalt, Zuckergehalt, Fettgehalt etc.) bestimmter Pflanzenbestandteile im Erntegutstrom, insbesondere des Korns.

Das optische Messsystem 9 weist einen ersten optischen Sensor 10 zur ortsaufgelösten Aufnahme von sichtbarem Licht aus einem sichtbaren Wellenlängenbereich in einem ersten Sichtfeld 11 auf. Aufgrund der zum Teil leicht voneinander abweichenden Definitionen des sichtbaren Wellenlängenbereichs wird hier unter sichtbarem Licht der Wellenlängenbereich zwischen 380 nm und 780 nm verstanden.

Der Begriff "Sichtfeld" betrifft jenen dreidimensionalen Raum, aus dem Licht über die entsprechende Optik auf den jeweiligen optischen Sensor treffen kann. Das Sichtfeld wird im allgemeinen Sprachgebrauch auch häufig mit dem englischen Begriff "Field of View" bezeichnet. Der Begriff "ortsaufgelöst" bedeutet vorliegend, dass das Sichtfeld des jeweiligen optischen Sensors in mehrere Teil-Sichtfelder unterteilt ist, die messtechnisch voneinander unterscheidbar sind. Der jeweilige optische Sensor weist also mindestens zwei Bildpunkte auf, die wenigstens teilweise ein unterschiedliches Teil-Sichtfeld aufweisen. Ein Bildpunkt ist dabei eine zweidimensionale Abbildung eines Teil-Sichtfelds. Bildpunkte werden im allgemeinen Sprachgebrauch häufig auch mit dem englischen Begriff "Pixel" bezeichnet. Wie ersichtlich ist, wird vorliegend eine weite Definition von Optik verwendet, die nicht nur den sichtbaren Wellenlängenbereich betrifft.

Das optische Messsystem 9 nimmt in einer Messroutine ortsaufgelöste Bilddaten des ersten optischen Sensors 10 in dem sichtbaren Wellenlängenbereich auf, die Erntegut 4 in einem ersten Ausschnitt A₁ des Erntegutstroms abbilden. Der Begriff "Bilddaten" ist hierbei weit zu verstehen und betrifft ganz allgemein Sensordaten eines optischen Sensors. Der Ausschnitt des Ernteguts 4 ist der vom optischen Sensor zum Zeitpunkt der Aufnahme aus sichtbare Teil des Ernteguts 4. Er betrifft also den im Sichtfeld befindlichen, nicht verdeckten Teil des Ernteguts 4.

Die Erntemaschine weist weiterhin eine Auswerteeinrichtung 12 zur Ermittlung eines, insbesondere die Zusammensetzung und/oder Inhaltsstoffe des Ernteguts 4 betreffenden, Erntegutparameters auf.

Wesentlich ist nun, dass das optische Messsystem 9 einen zweiten optischen Sensor 13 zur Aufnahme von nicht-sichtbarem Licht aus einem nicht-sichtbarem Wellenlängenbereich in einem zweiten Sichtfeld 14 aufweist. Der jeweilige optische Sensor 10, 13 muss dabei nicht ausnahmslos sichtbares bzw. nicht-sichtbares Licht aufnehmen. Gewisse Überlappungen sind möglich. Es müssen jedoch mehr als 50% des aufgenommenen Lichts der aufzunehmenden Wellenlängenbereiche des jeweiligen optischen Sensors 10, 13 aus dem sichtbaren bzw. nicht-sichtbaren Bereich stammen. Vorzugsweise müssen sogar mindestens 90%, weiter vorzugsweise mindestens 99%, aus dem jeweiligen Wellenlängenbereich stammen.

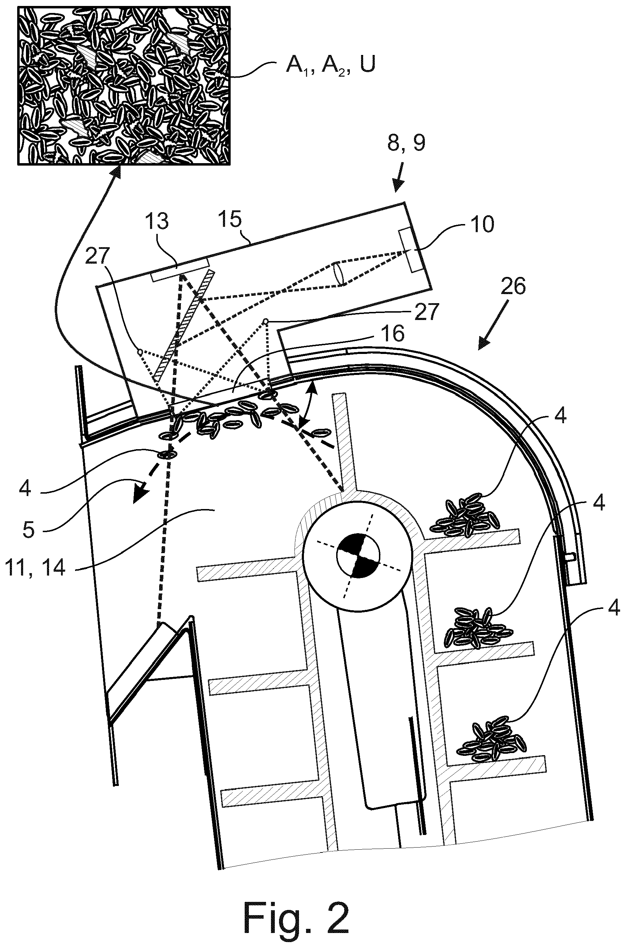

In Fig. 2 sind der erste und der zweite optische Sensor 10, 13 durch einen teildurchlässigen Spiegel getrennt dargestellt. Diese Möglichkeit ist jedoch nur beispielhaft zu verstehen.

In der Messroutine nimmt das optische Messsystem 9 Bilddaten des zweiten optischen Sensors 13 in dem nicht-sichtbaren Bereich auf, die Erntegut 4 in einem zweiten Ausschnitt A₂ des Erntegutstroms abbilden.

Wesentlich ist weiterhin, dass der erste und der zweite Ausschnitt A_{1,} A₂ zumindest teilweise in einem Überlappungsausschnitt U überlappen und dass die Auswerteeinrichtung 12 in einer Analyseroutine die Bilddaten des ersten optischen Sensors 10 zu dem Überlappungsausschnitt U und des zweiten optischen Sensors 13 zu dem Überlappungsausschnitt U miteinander korreliert und so den Erntegutparameter ermittelt.

Der Überlappungsausschnitt U kann dabei zeitlich und/oder örtlich gebildet werden. Beispielsweise können der erste optische Sensor 10 und der zweite optische Sensor 13 örtlich versetzt zueinander angeordnet sein. Da hier und vorzugsweise der Auswerteeinrichtung 12 eine Geschwindigkeit des Erntegutstroms bekannt ist, kann so durch zeitlichen Versatz der Aufnahme der Überlappungsausschnitt U rechnerisch gebildet werden. Alternativ kann der Überlappungsausschnitt U örtlich gebildet werden, also durch ein Überlappen des ersten und zweiten Sichtfeldes 11, 14.

Es ergibt sich von selbst, dass der Ernteguttransportweg 5 jedenfalls teilweise durch das erste und durch das zweite Sichtfeld 11, 14 verläuft. Hier und vorzugsweise betrifft der Überlappungsausschnitt U den Erntegutstrom und insbesondere den Haupterntegutstrom. Eine statische Messung ist jedoch ebenso vorteilhaft denkbar.

Der Begriff "korrelieren" ist hier weit zu verstehen. Ganz grundsätzlich werden die Bilddaten des ersten optischen Sensors 10 und des zweiten optischen Sensors 13 zusammen zur Ermittlung des Erntegutparameters herangezogen und dabei gemeinsam zur Ermittlung des Erntegutparameters verarbeitet. Hierfür können beispielsweise experimentell Abhängigkeiten zwischen den Bilddaten bestimmt werden.

Es ist hier und vorzugsweise vorgesehen, dass der erste optische Sensor 10 als Kamera, insbesondere als RGB-Kamera, ausgestaltet ist. Eine RGB-Kamera ist eine Farbkamera, die mindestens einen roten, mindestens einen grünen und mindestens einen blauen Farbkanal aufweist. Sie kann also drei unterscheidbare Wellenlängenbereiche aufnehmen. Dabei ist es üblicherweise so, dass die Sensorelemente der Kamera mittels eines Farbfilters, insbesondere nach Art eines Bayer-Patterns, auf die Wellenlängenbereiche aufgeteilt werden.

Der zweite optische Sensor 13 ist hier und vorzugsweise als Spektrometer, insbesondere als Nah-Infrarot-Spektrometer ausgestaltet. Ein Nah-Infrarot-Spektrometer kann mindestens zwei unterscheidbare Wellenlängenbereiche im nah-infraroten Bereich unterscheiden und eine Intensität des Lichts in diesem Wellenlängenbereich ermitteln. Hier und vorzugsweise ist der zweite optische Sensor 13 als optisches Spektrometer ausgestaltet.

Der nah-infrarote Wellenlängenbereich wird hier als von 780 nm bis 3000 nm verlaufend definiert. Der nah-ultraviolette Wellenlängenbereich wird hier als von 315 nm bis 380 nm verlaufend definiert.

Hier und vorzugsweise weist das optische Messsystem 9 ein Gehäuse 15 auf. Hier und vorzugsweise sind der erste optische Sensor 10 und der zweite optische Sensor 13 in dem Gehäuse 15 angeordnet. Dies betrifft hier und vorzugsweise jeweils den gesamten Sensor 10, 13. Alternativ kann auch nur ein Teil des jeweiligen optischen Sensors 10, 13 in dem Gehäuse 15 angeordnet sein, insbesondere können alle lichtaufnehmenden Sensorelemente des jeweiligen optischen Sensors 10, 13 in dem Gehäuse 15 angeordnet sein.

Das optische Messsystem 9 kann ein lichtdurchlässiges Fenster 16 aufweisen. Hier und vorzugsweise verläuft dann das von dem Erntegut 4, also von dem jeweiligen Ausschnitt A_{1,} A₂ des Ernteguts 4, ausgehende, von dem ersten und zweiten optischen Sensor 10, 13 aufgenommene Licht durch das lichtdurchlässige Fenster 16 und vorzugsweise von dem lichtdurchlässigen Fenster 16 bis zu dem ersten und zweiten optischen Sensor 10, 13 vollständig innerhalb des Gehäuses 15. Dies ist in Fig. 2 dargestellt. Es ist entsprechend bevorzugt, dass das lichtdurchlässige Fenster 16 Teil des Gehäuses 15 ist. Das lichtdurchlässige Fenster 16 kann in Kontakt mit dem Erntegut 4 kommen.

Die Ermittlung des Erntegutparameters kann derart ablaufen, dass die Auswerteeinrichtung 12 aus den Bilddaten des zweiten optischen Sensors 13 einen den Überlappungsausschnitt U betreffenden Erntegutparameter ermittelt, dass die Auswerteeinrichtung 12 aus den Bilddaten des ersten optischen Sensors 10 einen Korrekturwert, insbesondere Korrekturfaktor, ermittelt und den Erntegutparameter mittels des Korrekturfaktors korrigiert. Der Erntegutparameter wird hier also speziell zu dem Überlappungsausschnitt U ermittelt. Dies ist insbesondere dann relevant, wenn der Erntegutparameter über den Erntegutstrom nicht homogen ist.

Der Erntegutparameter und/oder der Korrekturwert können einen Kornanteil und/oder einen Bruchkornanteil und/oder einen Nicht-Kornanteil, insbesondere einen Anteil an Ährenspitzen und/oder unausgedroschenen Bestandteilen und/oder Stroh und/oder Stängelstücken und/oder Lieschblättern, und/oder einen Inhaltsstoff des Ernteguts 4, insbesondere einen Proteingehalt und/oder Stärkegehalt und/oder eine Kornfeuchtigkeit, betreffen. Der Korrekturwert kann eine Vermessbarkeit des ersten und/oder zweiten Ausschnitts A_{1,} A₂ durch den zweiten optischen Sensor 13 abbilden. Beispielsweise ist denkbar, dass mittels des zweiten optischen Sensors 13 ein Erntegutparameter gut ermittelbar ist, jedoch hinsichtlich eines Störfaktors, beispielsweise eines Nicht-Kornanteils, anfällig ist, der wiederum mittels des ersten optischen Sensors 10 gut ermittelbar ist. Wenn dann näherungsweise der Einfluss des Nicht-Kornanteils auf das Gesamtmessergebnis des zweiten Sensors 13 bekannt ist, kann dessen Messwert entsprechend korrigiert werden und somit die Vermessbarkeit des Ausschnitts berücksichtigt werden. Der Begriff "Vermessbarkeit" betrifft dabei die Frage, inwiefern eine Messung des Erntegutparameters in dem jeweiligen Ausschnitt A_{1,} A₂ durch den zweiten optischen Sensor 13 vollständig oder nur teilweise sinnvoll möglich ist.

Eine andere Möglichkeit ist, dass mittels der beiden optischen Sensoren 10, 13 diverse Erntegutparameter und Störfaktoren bei verschiedenen Wellenlängenbereichen unterschiedlich genau und sich gegenseitig störend ermittelt werden, wodurch ein Gleichungssystem gebildet wird, in dem die unterschiedlichen Faktoren jeweils zusammen das Messergebnis des jeweiligen Wellenlängenbereichs bilden. Bei ausreichender Bestimmtheit kann dieses Gleichungssystem dann gelöst werden. Interessant ist dabei auch, dass unterschiedliche Wellenlängen eine unterschiedliche Eindringtiefe in das Erntegut 4 aufweisen können, wodurch entsprechend unterschiedliche Erntegutparameter gemessen werden können.

Hier und vorzugsweise, wie mittels der beispielhaften RGB-Kamera angedeutet, kann der erste optische Sensor 10 zur ortsaufgelösten Aufnahme von sichtbarem Licht aus mindestens zwei, vorzugsweise mindestens drei, unterscheidbaren sichtbaren Wellenlängenbereichen in dem ersten Sichtfeld 11 eingerichtet sein. Der erste optische Sensor 13 kann zusätzlich oder alternativ zur ortsaufgelösten Aufnahme von sichtbarem Licht des gesamten sichtbaren Wellenlängenbereichs in dem ersten Sichtfeld 11 eingerichtet sein. Die unterscheidbaren Wellenlängenbereiche können gleichzeitig oder sequentiell aufgenommen werden. Das gleichzeitige Aufnehmen kann mittels eines Bayer-Patterns, Lichtbrechung, Strahlteilung und dergleichen erreicht werden. Das sequentielle Aufnehmen kann passiv durch Filterwechsel nach Art eines Filterrades oder durch sequentielle Beleuchtung in den unterschiedlichen Wellenlängenbereichen aktiv erreicht werden.

Der erste optische Sensor 10 kann als Zeilenkamera oder Flächenkamera mit Sensorelementen ausgestaltet sein. Die Sensorelemente nehmen jeweils örtlich unterschiedliche, insbesondere voneinander beabstandete, Bildpunkte des ersten Sichtfelds 11 auf. Es ist dabei bevorzugt so, dass die Bildpunkte einander nicht überlappen. Vorzugsweise weist der erste optische Sensor 10 mindestens 1.000, weiter vorzugsweise mindestens 10.000, Sensorelemente auf, die jeweils den gleichen Wellenlängenbereich aufnehmen. Der erste optische Sensor 10 weist also beispielsweise 1.000.000 mit einem grünen Filter versehene Sensorelemente auf. Zusätzlich oder alternativ können die Sensorelemente flächig, insbesondere auf einen gemeinsamen Sensorchip, angeordnet sein. Die Sensorelemente können nach bekannten Techniken beispielsweise als CCD-Sensorelemente oder CMOS-Sensorelemente oder InGaAs-Sensorelemente ausgestaltet sein. Sie können also, je nach zählweise einzeln oder in, insbesondere Vierer-, Gruppen, das bilden, was üblicherweise ebenfalls mit dem englischen Begriff "Pixel" bezeichnet wird.

Es lässt sich zusammenfassend sagen, dass der erste optische Sensor 10 hier und vorzugsweise für die ortsaufgelöste Aufnahme weniger Spektralbereiche vorgesehen ist. Dabei ist hier und vorzugsweise der Ortsauflösung jeweils auch eine Spektralauflösung zugeordnet, so dass sich aus Ortsauflösung und Spektralauflösung eine Matrix ergibt. Dies ist bekanntermaßen bei einem Bayer-Pattern der Fall.

Für den zweiten optischen Sensor 13 lässt sich zusammenfassend vorwegnehmen, dass dieser vorzugsweise eine geringe Ortsauflösung und eine prinzipiell beliebige spektrale Auflösung aufweist.

Der zweite optische Sensor 13 ist vorzugsweise zur Aufnahme von nicht-sichtbarem Licht aus mindestens zwei, vorzugsweise mindestens 100, weiter vorzugsweise mindestens 1000, noch weiter vorzugsweise mindestens 5000 und/oder höchstens 20000, vorzugsweise höchstens 10000 unterscheidbaren Wellenlängenbereichen eingerichtet. Der zweite optische Sensor 13 weist zusätzlich oder alternativ höchstens 1.000, vorzugsweise höchstens 500, weiter vorzugsweise höchstens 100, weiter vorzugsweise höchstens 10, insbesondere keine, Sensorelemente auf, die örtlich unterschiedliche Bildpunkte aufnehmen. Der zweite Sensor 13 ist hier und vorzugsweise als, insbesondere nicht-ortsaufgelöstes, Spektrometer ausgestaltet. Es kann also vorgesehen sein, dass der zweite optische Sensor 13 pro spektralem Bildpunkt nur genau einen örtlichen Bildpunkt aufweist.

Es kann vorgesehen sein, dass der erste optische Sensor 10 mindestens doppelt so viele Sensorelemente, vorzugsweise mindestens viermal so viele Sensorelemente, weiter vorzugsweise mindestens zehnmal so viele Sensorelemente, weiter vorzugsweise mindestens hundertmal so viele Sensorelemente, wie der zweite optische Sensor 13 aufweist.

Hier und vorzugsweise ist mindestens ein Sensorelement des zweiten optischen Sensors 13 zur Aufnahme von nicht-sichtbarem Licht aus mindestens einem Wellenlängenbereich umfassend 1000 nm und/oder 1200 nm bis 1800 nm und/oder 2000 nm und/oder umfassend 2100 nm und/oder 2200 nm und/oder 2300 nm und/oder 2400 nm und/oder 2500 nm und/oder umfassend 2200 bis 2400 nm und/oder umfassend 2100 bis 2500 nm und/oder umfassend einen Teil des nah-infraroten Spektrums und/oder umfassend einen Teil des nah-ultravioletten Spektrums ausgebildet.

Weiterhin kann mindestens ein Sensorelement des zweiten optischen Sensors 13 zur Aufnahme von nicht-sichtbarem Licht aus mindestens einem Wellenlängenbereich umfassend 300 nm und/oder 330 nm und/oder 350 nm und/oder 380 nm, ausgebildet sein.

Ein weiterer im landwirtschaftlichen Bereich interessanter Wellenlängenbereich liegt um 405 nm. Bei dieser Wellenlänge ist insbesondere die Erkennung des Bruchkornanteils besonders einfach möglich. Es kann auch vorgesehen sein, dass der zweite optische Sensor 13 zusätzlich zu einem nicht-sichtbaren Wellenlängenbereich zur Aufnahme von Licht aus einem Wellenlängenbereich umfassend 405 nm ausgebildet ist.

Die unterscheidbaren Wellenlängenbereiche des zweiten optische Sensors 13 sind hier und vorzugsweise breiter als 0,1 nm, vorzugsweise breiter als 1 nm, weiter vorzugsweise breiter als 4 nm und/oder weniger breit als 100 nm, vorzugsweise weniger breit als 50 nm, weiter vorzugsweise weniger breit als 10 nm, noch weiter vorzugsweise weniger breit als 5 nm.

Hier und vorzugsweise deckt der erste Ausschnitt A₁ mindestens 50%, weiter vorzugsweise mindestens 90%, noch weiter vorzugsweise mindestens 95%, noch weiter vorzugsweise mindestens 99% und noch weiter vorzugsweise 100% des zweiten Ausschnitts A₂ ab. Das Abdecken ist dabei zeitlich und/oder örtlich gemeint.

Alternativ kann bezüglich der zeitlichen Überdeckung vorgesehen sein, dass der erste Ausschnitt A₁ und der zweite Ausschnitt A₂ an ihren Rändern überlappen oder höchstens einen Meter, vorzugsweise höchstens einen halben Meter, weiter vorzugsweise höchstens zehn Zentimeter voneinander beabstandet sind.

Bei dem hier dargestellten Mähdrescher 1 zählt zu den Arbeitsaggregaten 2 neben dem bereits erwähnten Schneidwerk 6 ein mit diesem verbundene Schrägförderer 17, von dem aus der Erntegutstrom an von einem Dreschkorb 18 ummantelte Dreschorgane 19 übergeben wird. Über eine Umlenktrommel 20 gelangt der Erntegutstrom in eine hier als Trennrotor ausgebildete Trenneinrichtung 21, in welcher freibewegliche Körner des Erntegutstroms in einem unteren Bereich abgeschieden werden. Von hier aus gelangt der Erntegutstrom über einen Rücklaufboden 22 zu einer Reinigungsvorrichtung 23, die wie hier dargestellt aus mehreren Siebebenen 24 und einem Gebläse 25 besteht. Von hier aus führt der Kornelevator 26 den Erntegutstrom schließlich zum Korntank 7. All diese Arbeitsaggregate 2 tragen dabei zur Verarbeitung des Ernteguts 4 bei.

Das optische Messsystem 9 ist hier und vorzugsweise an dem Kornelevator 26 angeordnet. Ganz allgemein kann das lichtdurchlässige Fenster 16 an den Ernteguttransportweg 5 angrenzen und/oder diesen begrenzen. Das lichtdurchlässige Fenster 16 kann für alle von dem optischen Messsystem 9 aufgenommenen Wellenlängenbereiche durchlässig sein. Das erste und das zweite Sichtfeld 11, 14 können vollständig durch das lichtdurchlässige Fenster 16 verlaufen.

Es kann vorgesehen sein, dass das optische Messsystem 9 eine Lichtquelle 27 aufweist. Die Lichtquelle 27 ist vorzugsweise eingerichtet, Licht einiger oder aller von dem optischen Messsystem 9 aufgenommene Wellenlängenbereiche gleichzeitig abzustrahlen. Zusätzlich oder alternativ kann die Lichtquelle 27 eingerichtet sein, sequentiell Licht einiger oder aller von dem optischen Messsystem 9 aufgenommene Wellenlängenbereiche abzustrahlen.

Hier und vorzugsweise bestrahlt die Lichtquelle 27 den Überlappungsausschnitt U und/oder den ersten Ausschnitt A₁ und/oder den zweiten Ausschnitt A₂. Vorzugsweise bestrahlt die Lichtquelle 27 den jeweiligen Ausschnitt A_{1,} A₂ aus Richtung des ersten und/oder zweiten optischen Sensors 10, 13. Es ist also bevorzugt, dass der jeweilige Sensor 10, 13 reflektiertes Licht anstelle von transmittiertem Licht aufnimmt. Insbesondere kann auch vorgesehen sein, dass die Lichtquelle 27 außerhalb des ersten und/oder zweiten Sichtfelds 11, 14 angeordnet ist.

Wie bereits erwähnt, weist die landwirtschaftliche Erntemaschine einen Kornelevator 26 auf. Hier und vorzugsweise ist der erste optische Sensor 10 und/oder der zweite optische Sensor 13 und/oder das lichtdurchlässige Fenster 16 und/oder die Lichtquelle 27 und/oder das Gehäuse 15 an, insbesondere unter oder über, dem Kornelevator 26 angeordnet. Alternativ kann der erste optische Sensor 10 und/oder der zweite optische Sensor 13 und/oder das lichtdurchlässige Fenster 16 und/oder die Lichtquelle 27 und/oder das Gehäuse 15 hinter dem Kornelevator 26, insbesondere im Bereich einer Korntankbefüllung, angeordnet sein. Weitere mögliche Anordnungen, insbesondere bei einem Feldhäcksler im Bereich eines Auswurfs, sind ebenso bevorzugt. Auch eine Anordnung vor dem Kornelevator 26 ist möglich. In dem letztgenannten Fall ist das oder sind die jeweiligen Elemente vorzugsweise hinter dem letzten dreschenden oder dem letzten abscheidenden Arbeitsaggregat 2 angeordnet.

Es kann entsprechend vorgesehen sein, dass der Überlappungsausschnitt U Teil einer Unterseite oder einer Oberseite des Erntegutstroms, insbesondere des Haupterntegutstroms, entlang des Ernteguttransportweges 5 ist.

Gemäß einer weiteren Lehre, der eigenständige Bedeutung zukommt, wird ein Verfahren zum Betrieb einer vorschlagsgemäßen landwirtschaftlichen Erntemaschine vorgeschlagen, bei dem mittels der landwirtschaftlichen Arbeitsmaschine ein Feldbestand 3 abgeerntet und Erntegut 4 verarbeitet wird, wobei mittels des optischen Messsystems 9 die Messroutine durchgeführt wird und wobei mittels der Auswerteinrichtung 12 die Analyseroutine durchgeführt wird. Auf alle Ausführungen zu der vorschlagsgemäßen landwirtschaftlichen Erntemaschine darf verwiesen werden.

Bevorzugt bei dem Verfahren ist, dass die Messroutine und die Analyseroutine mehrfach, insbesondere fortlaufend, durchgeführt werden. Vorzugsweise wird der Erntegutparameter in Echtzeit ermittelt. Der Begriff "Echtzeit" bedeutet hier, dass zwischen Aufnahme der jeweiligen Bilddaten und Ermittlung des Erntegutparameters nur eine vordefinierte Zeitspanne liegt, die hier und vorzugsweise kleiner als eine Minute, vorzugsweise kleiner als 30 Sekunden, weiter vorzugsweise kleiner als fünf Sekunden ist. Der Erntegutparameter kann einem Benutzer angezeigt werden.

Insbesondere im Rahmen des vorschlagsgemäßen Verfahrens kann vorgesehen sein, dass die Kontrollanordnung 8 zyklisch Bildserien aufnimmt. Vorzugsweise wird dann innerhalb einer vorbestimmten Verarbeitungszeit nach der Aufnahme einer Bildserie ein auf der Bildserie basierender Erntegutparameter ermittelt und angezeigt.

### Bezugszeichenliste

- 1: Mähdrescher
- 2: Arbeitsaggregat
- 3: Feldbestand
- 4: Erntegut
- 5: Ernteguttransportweg
- 6: Schneidwerk
- 7: Korntank
- 8: Kontrollanordnung
- 9: optisches Messsystem
- 10: erster optischer Sensor
- 11: erstes Sichtfeld
- 12: Auswerteeinrichtung
- 13: zweiter optischer Sensor
- 14: zweites Sichtfeld
- 15: Gehäuse
- 16: lichtdurchlässiges Fenster
- 17: Schrägförderer
- 18: Dreschkorb
- 19: Dreschorgane
- 20: Umlenktrommel
- 21: Trenneinrichtung
- 22: Rücklaufboden
- 23: Reinigungsvorrichtung
- 24: Siebebenen
- 25: Gebläse
- 26: Kornelevator
- 27: Lichtquelle
- A₁: erster Ausschnitt
- A₂: zweiter Ausschnitt
- U: Überlappungsausschnitt

## Patentansprüche

1. Landwirtschaftliche Erntemaschine, insbesondere Mähdrescher (1) oder Feldhäcksler, mit mindestens einem Arbeitsaggregat (2) zum Abernten eines Feldbestandes (3) und zur Verarbeitung von Erntegut (4) aus dem Feldbestand (3), wobei das Erntegut (4) im Betrieb der Erntemaschine entlang eines Ernteguttransportweges (5) durch die Erntemaschine in einem Erntegutstrom transportiert wird,
mit einer Kontrollanordnung (8), die ein an dem Ernteguttransportweg (5) angeordnetes optisches Messsystem (9) zur Analyse der Zusammensetzung und/oder von Inhaltsstoffen des Ernteguts (4) aufweist,
wobei das optische Messsystem (9) einen ersten optischen Sensor (10) zur ortsaufgelösten Aufnahme von sichtbarem Licht aus einem sichtbaren Wellenlängenbereich in einem ersten Sichtfeld (11) aufweist, wobei das optische Messsystem (9) in einer Messroutine ortsaufgelöste Bilddaten des ersten optischen Sensors (10) in dem sichtbaren Wellenlängenbereich aufnimmt, die Erntegut (4) in einem ersten Ausschnitt (A₁) des Erntegutstroms abbilden,
wobei die Erntemaschine eine Auswerteeinrichtung (12) zur Ermittlung eines, insbesondere die Zusammensetzung und/oder Inhaltsstoffe des Ernteguts (4) betreffenden, Erntegutparameters aufweist,
**dadurch gekennzeichnet,**
**dass** das optische Messsystem (9) einen zweiten optischen Sensor (13) zur Aufnahme von nicht-sichtbarem Licht aus einem nicht-sichtbaren Wellenlängenbereich in einem zweiten Sichtfeld (14) aufweist, dass das optische Messsystem (9) in der Messroutine Bilddaten des zweiten optischen Sensors (13) in dem nicht-sichtbaren Wellenlängenbereich aufnimmt, die Erntegut (4) in einem zweiten Ausschnitt (A₂) des Erntegutstroms abbilden,
**dass** der erste und der zweite Ausschnitt (A_{1,} A₂) zumindest teilweise in einem Überlappungsausschnitt (U) überlappen und dass die Auswerteeinrichtung (12) in einer Analyseroutine die Bilddaten des ersten optischen Sensors (10) zu dem Überlappungsausschnitt (U) und des zweiten optischen Sensors (13) zu dem Überlappungsausschnitt (U) miteinander korreliert und so den Erntegutparameter ermittelt.

2. Landwirtschaftliche Erntemaschine nach Anspruch 1, **dadurch gekennzeichnet, dass** der erste optische Sensor (10) als Kamera, insbesondere RGB-Kamera, ausgestaltet ist, und/oder, dass der zweite optische Sensor (13) als, insbesondere Nah-Infrarot-, Spektrometer ausgestaltet ist.

3. Landwirtschaftliche Erntemaschine nach Anspruch 1 oder 2, **dadurch gekennzeichnet, dass** das optische Messsystem (9) ein Gehäuse (15) aufweist und der erste optische Sensor (10) und der zweite optische Sensor (13) in dem Gehäuse (15) angeordnet sind, vorzugsweise, dass das optische Messsystem (9) ein lichtdurchlässiges Fenster (16) aufweist und das von dem Erntegut (4) ausgehende, von dem ersten und zweiten optischen Sensor (10, 13) aufgenommene Licht durch das lichtdurchlässige Fenster (16) und von dem lichtdurchlässigen Fenster (16) bis zu dem ersten und zweiten optischen Sensor (10, 13) vollständig innerhalb des Gehäuses (15) verläuft.

4. Landwirtschaftliche Erntemaschine nach einem der vorhergehenden Ansprüche, **dadurch gekennzeichnet, dass** die Auswerteeinrichtung (12) in der Analyseroutine aus den Bilddaten des zweiten optischen Sensors (13) einen den Überlappungsausschnitt (U) betreffenden Erntegutparameter ermittelt, dass die Auswerteeinrichtung (12) aus den Bilddaten des ersten optischen Sensors (10) einen Korrekturwert, insbesondere Korrekturfaktor, ermittelt und den Erntegutparameter mittels des Korrekturfaktors korrigiert.

5. Landwirtschaftliche Erntemaschine nach einem der vorhergehenden Ansprüche, **dadurch gekennzeichnet, dass** der Erntegutparameter und/oder der Korrekturwert einen Kornanteil und/oder einen Bruchkornanteil und/oder einen Nicht-Kornanteil, insbesondere einen Anteil an Ährenspitzen und/oder unausgedroschenen Bestandteilen und/oder Stroh und/oder Stängelstücken und/oder Lieschblättern, und/oder einen Inhaltsstoff des Ernteguts (4), insbesondere einen Proteingehalt und/oder einen Stärkegehalt, und/oder eine Kornfeuchtigkeit, betrifft, und/oder, dass der Korrekturwert eine Vermessbarkeit des ersten und/oder zweiten Ausschnitts (A1, A2) durch den zweiten optischen Sensor (13) abbildet.

6. Landwirtschaftliche Erntemaschine nach einem der vorhergehenden Ansprüche, **dadurch gekennzeichnet, dass** der erste optische Sensor (10) zur ortsaufgelösten Aufnahme von sichtbarem Licht aus mindestens zwei, vorzugsweise mindestens drei, unterscheidbaren sichtbaren Wellenlängenbereichen in dem ersten Sichtfeld (11) eingerichtet ist, und/oder, dass der erste optische Sensor (10) zur ortsaufgelösten Aufnahme von sichtbarem Licht des gesamten sichtbaren Wellenlängenbereichs in dem ersten Sichtfeld (11) eingerichtet ist.

7. Landwirtschaftliche Erntemaschine nach einem der vorhergehenden Ansprüche, **dadurch gekennzeichnet, dass** der erste optische Sensor (10) als Zeilenkamera oder Flächenkamera mit Sensorelementen ausgestaltet ist, dass die Sensorelemente jeweils örtlich unterschiedliche, insbesondere voneinander beabstandete, Bildpunkte des ersten Sichtfelds (11) aufnehmen.

8. Landwirtschaftliche Erntemaschine nach einem der vorhergehenden Ansprüche, **dadurch gekennzeichnet, dass** der zweite optische Sensor (13) höchstens 1000, vorzugsweise höchstens 500, weiter vorzugsweise höchstens 100, weiter vorzugsweise höchstens 10, insbesondere keine, Sensorelemente aufweist, die örtlich unterschiedliche Bildpunkte aufnehmen, vorzugsweise, dass der zweite optische Sensor (13) als nicht-ortsaufgelöstes Spektrometer ausgestaltet ist.

9. Landwirtschaftliche Erntemaschine nach einem der vorhergehenden Ansprüche, **dadurch gekennzeichnet, dass** mindestens ein Sensorelement des zweiten optischen Sensors (13) zur Aufnahme von nicht-sichtbarem Licht aus mindestens einem Wellenlängenbereich umfassend 1000 nm und/oder 1200 nm bis 1800 nm und/oder 2000 nm und/oder umfassend 2100 nm und/oder 2200 nm und/oder 2300 nm und/oder 2400 nm und/oder 2500 nm, und/oder umfassend 2200 bis 2400 nm und/oder umfassend 2100 bis 2500 nm und/oder umfassend einen Teil des nah-infraroten Spektrums und/oder umfassend einen Teil des nah-ultravioletten Spektrums ausgebildet ist, und/oder, dass mindestens ein Sensorelement des zweiten optischen Sensors (13) zur Aufnahme von nicht-sichtbarem Licht aus mindestens einem Wellenlängenbereich umfassend 300 nm und/oder 330 nm und/oder 350 nm und/oder 380 nm, ausgebildet ist.

10. Landwirtschaftliche Erntemaschine nach einem der vorhergehenden Ansprüche, **dadurch gekennzeichnet, dass** der erste Ausschnitt (A₁) mindestens 50%, weiter vorzugsweise mindestens 90%, noch weiter vorzugsweise mindestens 95%, noch weiter vorzugsweise mindestens 99%, noch weiter vorzugsweise 100%, des zweiten Ausschnitts (A₂) abdeckt.

11. Landwirtschaftliche Erntemaschine nach einem der Ansprüche 3 bis 10, **dadurch gekennzeichnet, dass** das lichtdurchlässige Fenster (16) an den Ernteguttransportweg (5) angrenzt und/oder diesen begrenzt, vorzugsweise, dass die landwirtschaftlichen Erntemaschine einen Kornelevator (26) aufweist und der erste optische Sensor (10) und/oder zweite optische Sensor (13) und/oder das lichtdurchlässige Fenster (16) und/oder die Lichtquelle (27) und/oder das Gehäuse (15) an, insbesondere über, dem Kornelevator (26) angeordnet ist.

12. Verfahren zum Betrieb einer landwirtschaftlichen Erntemaschine nach einem der vorhergehenden Ansprüche, bei dem mittels der landwirtschaftlichen Arbeitsmaschine ein Feldbestand (3) abgeerntet und Erntegut (4) verarbeitet wird, wobei mittels des optischen Messsystems (9) die Messroutine durchgeführt wird und wobei mittels der Auswerteinrichtung (12) die Analyseroutine durchgeführt wird.
